(19)

Europäisches
Patentamt
European
Patent Office
Office européen
des brevets

(11) **EP 1 982 645 A1**

(12) **EUROPEAN PATENT APPLICATION**

(43) Date of publication:
**22.10.2008 Bulletin 2008/43**

(51) Int Cl.:
***A61B 5/00*** *(2006.01)*     ***A61B 19/00*** *(2006.01)*

(21) Application number: **08007595.5**

(22) Date of filing: **18.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **19.04.2007 DE 102007018641**

(71) Applicant: **Carl Zeiss Surgical GmbH
73447 Oberkochen (DE)**

(72) Inventors:
• **Hauger, Christoph
73431 Aalen (DE)**
• **Nahm, Werner
74426 Bühlerzell (DE)**

(74) Representative: **Diehl & Partner
Patentanwälte
Augustenstrasse 46
80333 München (DE)**

(54) **Method and apparatus for displaying a field of a brain of a patient and navigation system for brain surgery**

(57)     A method and an apparatus for displaying a part of a brain of a patient is provided. Laser-Doppler-images are acquired before and after a stimulation. The laser-Doppler-images comprise information about a blood motion or about a perfusion at the acquired part. Then, the blood motion images acquired before and after the stimulation are compared to identify areas in the brain of the patient which are related to the stimulation.

Fig. 1

EP 1 982 645 A1

**Description**

This invention relates to a method and an apparatus for displaying a field of a brain of a patient. In particular, this invention relates to a method for determining a continuous field in the brain which is activated or deactivated after a stimulation. Further, the invention relates to a navigation system for brain surgery which is suitable for displaying a field of the brain and determining a continuous field of the brain.

From the prior art a variety of methods for examining the brain are known. Image-forming methods, that means methods which display extended areas of the brain are for example computer tomography (CT), magnetic resonance tomography (MRT) and ultrasonic methods. As methods for functionally examining the brain are known for example electro encephalography (EEG), magneto encephalography (MEG), positron emission tomography (PET) as well as functional magnetic resonance tomography (fMRT). In recent years in particular the functional magnetic resonance tomography (fMRT) contributed to a large extend to the understanding of the construction of the brain. A neuronal activation of certain brain areas may cause an increase of the oxidised haemoglobin in the capillaries of the activated brain tissue. Using fMRT it is possible to locally detect the increase of the oxidised haemoglobin. A contrast due to a degree of oxygenation of the blood results from a changed susceptibility of the blood in dependence of the degree of oxygenation. This effect is referred to as BOLD (Blood Oxygenation Level Dependent)-contrast. With this technique it is thus possible to measure a brain activation of certain areas in the brain with a time resolution of a few seconds and thus to identify functional areas in the brain. A disadvantage of this technique is the relatively coarse time resolution and the high costs. Also, due to the relatively large required integration times of the measurement, it is necessary that the patient does not move during this time span. Otherwise, due to movements of the patient, massive artefacts arise in the functional information which can later only hardly be corrected. For this, namely elaborate rotations and translations of the data sets relative to each other are necessary which may even comprise deformations of the data sets. In spite of these problems and of these disadvantages of fMRT this technique is to date the method of choice to identify functional areas in the brain. Thereby, typically fMRT-images before and after a stimulation are compared. From the differences between both images areas of the brain relating to the stimulation may be determined. In particular, these examinations discovered that an increased activity of the brain causes an increased blood flow and an increased oxygen concentration.

An object of the present invention is to provide an improved method for identifying functional areas in the brain of a patient as well as to provide an apparatus for carrying out this method. A further object of the present invention is to provide an apparatus for brain surgeries.

According to a first aspect of the present invention a method for displaying a field of a brain of a patient is provided, wherein the method comprises: illuminating the field of the brain with measuring light and acquiring at least one first sequence of images of the illuminated field, then stimulating the patient, then illuminating the field of the brain with measuring light and acquiring at least one second sequence of images of the illuminated field, and evaluating the first and the second sequence of images by: (a) associating a first analysis image value to pixels in the first sequence of images to which pixels same locations of the field are imaged, respectively, wherein the first analysis image value depends on at least one of amplitudes and frequencies of temporal changes of the image values of these pixels, (b) associating a second analysis image value to pixels in the second sequence of images to which pixels the same locations of the field are imaged, respectively, wherein the second analysis image value depends on at least one of amplitudes and frequencies of temporal changes of image values of these pixels, and wherein the method further comprises: determining an output image from the first and the second analysis image values and displaying the output image.

During performing this method typically the skullcap of the patient is opened. The first sequence of images acquires a reference state of the field of the brain. Then the patient is stimulated to provoke an activation of certain areas of the brain. The subsequently acquired second sequence of images may represent an activated state of the field of the brain. An image is given by image values in a plurality of pixels. The image values thereby correspond to detected intensities of measuring light which has interacted with the field of the brain and is returned from there. Image values of pixels are analysed with respect to their temporal changes. In particular, at least one of amplitudes and frequencies of these temporal changes are used to determine for the first sequence of images a first analysis image value and to determine for the second sequence of images a second analysis image value. An output image is determined from first and second analysis image values and the output image is subsequently displayed. Thereby, the displaying may be carried out in the form of a two dimensional image, a diagram, a plurality of diagrams and the like. The two dimensional image may be a grey scale image, a pseudo colour image, or the like. Also three dimensional illustrations may be employed. A dependency of the first and second analysis image value on at least one of amplitudes and frequencies of temporal changes of image values is present if the first, respectively second, analysis image value changes due to temporal changes of the first, respectively second, image values. Thus, hereby, a constant dependency is not meant. The output image is thereby determined from first and second analyses image values, so that the output image changes, if either the first or the second analysis image values change.

According to an embodiment of the invention the acquiring the images of the first sequence and the second sequence comprises exposing detector elements of an image detector during an exposure time of less than 1 ms, in particular less than 0.1 ms, and further in particular less than 0.03 ms. Restricting the exposure time results in accumulating the light emanating from the illuminated field of the brain only over short times of less than 1 ms to detect image values. Thus, temporal fluctuations of high frequencies can be detected which is necessary for performing the method.

According to a further embodiment of the method the acquiring the images of the first and the second sequence comprises acquiring temporally subsequent images having a temporal interval of less than 1 ms, in particular less than 0.1 ms, and further in particular less than 0.05 ms. By choosing these temporal intervals between subsequent images it is possible, to detect frequencies of temporal changes of image values which comprise 0.5 kHz, 5 kHz and in particular 10 kHz. Thus, intensity fluctuations are detectable which result from Laser-Doppler-Signals which are generated from moving particles having a velocity between 0.4 mm/s and 8 mm/s, when light of a wavelength of about 800 nm is used as illumination light.

According to a further embodiment of the method the acquiring the images comprises detecting measuring light, wherein intensities of the detected measuring light having wavelengths greater than 1300 nm and smaller than 600 nm, in particular greater than 820 nm and smaller than 780 nm, amount to less than 10 % of a total intensity of the detected measuring light. Using light having wavelengths of a thus defined range enables a penetration depth into human tissue, in particular brain tissue, of about 1 mm to 2 mm. In particular, the wavelength range between 600 nm and 1300 nm is referred to as "diagnostic window". Thus, Laser-Doppler-Signals of particles which are present within this penetration depths and which move relative to each other can be detected. Thereby, the measuring light may exhibit a coherence length which substantially corresponds to twice the penetration depth of the measuring light into a tissue of the brain visible within the illuminated field. Using measuring light having this coherence length enables to detect an interference of light which was reflected by two particles which are a distance apart from each other corresponding to the penetration depth. Thus, it can be ensured that even the light penetrated in deepest locations of the field of the brain may interfere with light which was reflected at the surface of the field of the brain. Thus, a sensitivity may be increased.

According to an embodiment of the method the determining the output image from the first and the second analysis image values comprises computing at least one of a difference and a ratio of first and second terms, wherein the first terms depend on the first analysis image values and the second terms depend on the second analysis image values. The output image may thus for example be obtained by forming a difference of first and second analysis image values multiplied with different or same factors. Further, also a mathematical function may be applied to the first and second analysis image values, before forming a difference is carried out. These mathematical functions comprise for example forming a logarithm, forming a power, forming a product and the like. Upon similar modifications of the first and second analysis image values a ratio thereof may be formed to obtain the output image. A particular simple variant to determine a difference of the field of the brain before and after the stimulating is to determine the difference between the first and second analysis image.

According to a preferred embodiment of the method a coordinate system is established relative to a head of the patient. Thus, a fixed relationship between locations in the field of the brain and pixels to which the light emanating from the field of the brain is imaged is given. The establishing a coordinate system may thereby comprise fixing a reference frame at the head of the patient. Thereby, the head of the patient may be held at at least three pins or pillows. The pins or pillows thereby contact the scull of the patient. Alternatively, the coordinate system may also be established in that predetermined reference locations at the head of the patient are detected. The detecting predetermined reference locations may thereby comprise optically detecting. Thereby, plural cameras may be provided to detect coordinates of at least three predetermined locations at the head of the patient. Further, the cameras are used, to determine coordinates of the apparatus for displaying a field of a brain.

According to this last mentioned embodiment, further, coordinates of locations of the field of the brain may be determined in the coordinate system, wherein the locations are imaged to selected pixels in the output image.

According to a preferred embodiment of the method the method further comprises determining at least one continuous region in the output image in which intensity values are greater than a threshold. Thus it is possible, to mark regions in the output image which for example show large differences of analysis image values before and after the stimulation. These regions in the output image may correspond thereby to activated or deactivated areas in the field of the brain. This corresponds to identifying functional areas in the brain.

According to a preferred embodiment the at least one determined region may be displayed in superposition with the output image. Possible illustrations thereby comprise encircling the determined region using a line, a colouring the determined region, or the like.

According to a preferred embodiment coordinates of locations which are imaged to the at least one determined region in the output image are determined, in the coordinate system. Thus, locations of functional areas in the brain of the patient relative to a coordinate system of the head can be determined.

According to a preferred embodiment of the invention at least one of the first analysis image value and the second analysis image value comprises a value representing one of a perfusion, a concentration, an average velocity, a parameter of a velocity distribution, in particular a standard deviation of the velocity distribution of particles moving relative to each other, in particular blood cells, and a combination thereof. Thus it is possible, to detect and to quantify a blood motion using the claimed method. The thus specified analysis image value may thereby be obtained using a frequency spectrum of the temporal changes of image values. In particular, the concentration of particles moving relative to each other may be obtained as a 0th moment of the frequency distribution. The perfusion may be obtained as a 1st moment of the frequency distribution. An average velocity or a parameter of the velocity distribution may be obtained as a ratio between the perfusion and the concentration of particles moving relative to each other.

According to a preferred embodiment of the inventive method the method further comprises repeating the acquiring the at least one of the first and the second sequence of images. Further, the repeatedly acquired sequences of images are evaluated as described above. Thus, temporal sequences of first and second analysis image values are obtained. This may represent a movie of first and second analysis image values. Using such a movie a temporal course of an activation or a deactivation of brain areas after a simulation may be inferred.

According to a preferred embodiment the temporal sequences of the first, respectively the second, analysis image values may be at least one of displayed and stored. Displaying the temporal sequences of the first, respectively second, analysis image values may thereby correspond to playing a movie of the first, respectively second, analysis image values. A storing the sequences of the first, respectively second, analysis image values may thereby also comprise a storing the coordinates of the field of the brain in the coordinate system of the head of the patient, as well as the storing a representation of the stimulation carried out between the first and second sequences.

According to a preferred embodiment of the method the stimulating comprises requesting the patient, to move a particular body part. This body part may for example be an extremity, such as a finger.

According to a preferred embodiment of the present invention the stimulating comprises applying an agent to the patient. Thereby, the agent may be incorporated or may be externally applied. The applying the agent may also comprise irradiating the patient.

According to a preferred embodiment of the invention the stimulating comprises applying of at least one of a visual stimulus, an acoustic stimulus, a gestation stimulus, an olfaction stimulus, and a tactition stimulus to the patient. Thereby, images, such as photographs, paintings, text, may for example be presented to the patient or sounds, such as music or speech, may be played.

Also different kinds of food or beverages may be tasted by the patient, by applying the food or the beverages to the tongue of the patient, in particular by an application device. Physical properties of these applied nourishments, such as their temperature, may be controlled. Further the patient may be allowed to smell a substance by bringing the substance in proximity to its nose or introducing the substance into the patient's nose, in particular by a technical appliance. Thereby olfactory receptor neurons may be activated or deactivated.

Further, the patient may be allowed to experience touch or a somatic sensation. For this, objects having different roughness of their surfaces, different surface shapes, or different temperatures may contact a portion of the skin of the patient or may be slid over portions of the skin of the patient applying a varying amount of pressure. In particular the effect of anesthetics may be probed by applying this kind of stimulus.

Also the stimulus may comprise a request to carry out a conscious action. This action may for example comprise moving a finger.

According to a preferred embodiment of the inventive method the method further comprises removing a portion of the brain in dependence of at least one of the output image, the determined coordinates, and the at least one continuous region in the output image. The portion of the brain may comprise several regions. Thereby, a predetermined continuous region in the field of the brain may for example be circumvented to gain access to regions of the brain which are to removed. The regions to be removed may thereby be situated in layers of the brain which are located in a depth which is deeper than the penetration depth of the measuring light.

According to a preferred embodiment of the inventive method the method further comprises acquiring an image of the field of the brain in a visible wavelength range. Further, the image in a visible wavelength region may be displayed in superposition with at least one of the first analysis image values, the second analysis image values, and the output image. Here, in particular blood vessels situated at the surface of the brain may be imaged and concurrently the determined blood flow characteristics may be displayed.

According to another aspect of the present invention a navigation system for brain surgery is provided which comprises a position acquisition apparatus to acquire coordinates of locations of a brain of a patient, and a measuring apparatus for acquiring images which is configured to illuminate a field of the brain with measuring light, to acquire at least one sequence of images of the illuminated field, and to process the sequence of images by associating an analysis image value to pixels in the sequence of images to which pixels same locations of the field are imaged, respectively, wherein the analysis image value depends on at least one of amplitudes and frequencies of temporal changes of image values of these pixels. On one hand with this system laser-Doppler-images of a field of a brain can be acquired. On the other hand the provided position acquisition apparatus enables to associate individual points in these images to locations of the brain. Thus, locations in the brain may be identified whose Laser-Doppler-Signals change after a simulation. The position acquisition apparatus may thereby be designed in different ways. One possibility is the provision of a head coupling system which is adapted to hold the head of the patient by at least three pins or pillows in a fixed position relative to an object plane of the measuring apparatus for acquiring images. The position acquisition apparatus may also be an optical position acquisition apparatus, wherein the coordinates of predetermined locations of the head of the patient are determined. Concurrently thereto the coordinates of predetermined locations of the measuring apparatus for acquiring images are determined. Thus, a relationship between a coordinate system of the measuring apparatus for acquiring images and a coordinate system of the brain of the patient can be determined. Thus, a mapping between locations of images acquired by the measuring apparatus and locations of the brain of the patient is given. The measuring apparatus for acquiring images is adapted to temporally subsequently acquire a sequence of images of the field of the brain. This acquired sequence of images is then evaluated by analysing intensity values of the light emanating from the field of the brain with respect to its temporal changes, wherein the intensity values are obtained in every pixel. An analysis image value obtained from this analysis of the temporal changes of image values may thereby obtained using a frequency spectrum which can be determined from the temporal changes of the image values. In particular, moments of the frequency spectrum of the temporal changes may thereby be comprised.

According to a preferred embodiment of this aspect of the present invention the measuring apparatus for acquiring images comprises a camera having an exposure integration time of less than 1 ms, in particular less than 0.1 ms, and further in particular less than 0.03 ms. The exposure integration time may be adjustable. Thus, a detectable frequency of a change of the temporal changes of the image values is greater than 1 kHz, in particular greater than 10 kHz, and further in particular greater than 30 kHz. According to a preferred embodiment of the present invention the measuring apparatus for acquiring images comprises a camera having a frame rate greater than 1 kHz, in particular greater than 10 kHz, and further in particular greater than 20 kHz. A frame-rate of the camera is a frequency indicating a number of images acquired by the camera in a certain time span. If the frame-rate is for example 10 kHz the camera is enabled to acquire 10,000 images in one second. Thus it is possible, to sample the temporal changes of the image values with a frequency which is higher than 1 kHz, in particular higher as 10 kHz, and further in particular higher than 20 kHz. In particular, detectors of the camera comprise CMOS-sensors. A frequency analysis of such an acquired signal of temporal changes allows determining frequency components contributing to the signal of the temporal changes. According to the Nyquist-Theorems thus frequency components may be determined whose frequencies are greater than 0.5 kHz, in particular greater than 5 kHz, and further in particular greater than 10 kHz. An analysis of the temporal changes of the image values with respect to the determining a frequency spectrum comprises thereby the determining the amplitudes and frequencies of the frequency components which, as a sum, result to the acquired temporal changes.

According to a preferred embodiment of this aspect of the invention a band-pass filter is arranged in a beam path upstream of the camera, wherein the transmission characteristics of the band-pass filter is adapted such that a transmission of light having wavelengths greater than 1300 nm and smaller than 600 nm, in particular greater than 820 nm and smaller than 780 nm, amounts to less than 10 % of a total transmission of the band-pass filter. This enables that only desired light emanating from the field of the brain is detected by the camera. Hereby it is for example possible to use as measuring light a small spectral range of visible light and to concurrently illuminate the field of the brain for visual inspection with white light (light comprising wavelength in the visible range) avoiding disturbing the measurement by this white light reflected at the field of the brain.

According to a preferred embodiment of the present invention the measuring apparatus comprises a light source having an emission spectrum formed such that an emitted intensity of light having wavelengths greater than 1300 nm and smaller than 600 nm, in particular greater than 820 nm and smaller than 780 nm, amounts to less than 10 % of a total intensity. When in this embodiment no further perturbation light is incident onto the field of the brain it is thus ensured that the camera detects only light within a particular wavelength range.

According to a preferred embodiment of this aspect of the present invention the navigation system further comprises an apparatus for acquiring images of the brain of the patient in a visible wavelength range. With this provision it is possible to acquire beside laser-Doppler-images also images in the visible wavelength range. This may be advantageous to associate details of the field of the brain recognizable in the visible wavelength range with details in the laser-Doppler-images. For example, a blood flow in blood vessels at the surface of the brain may thus be examined. In particular, thereby also a blood flow may be examined in areas not pervaded by blood vessels recognizable in the visible wavelength range.

According to a preferred embodiment of this aspect of the present invention the navigation system further comprises a display apparatus for displaying an image in the visible wavelength range, in particular in superposition with analysis image values. Thus, it is further enabled to superimpose details recognizable in the visible wavelength range with details recognizable in the laser-Doppler-images. This facilitates an interpretation of the laser-Doppler-images by an observer.

According to a preferred embodiment of this aspect of the invention the navigation system is adapted to perform methods of the first aspect of the present invention. Thus, with the navigation system, laser-Doppler-images may be acquired before and after a stimulation and may be displayed and their changes may be determined. Thus, this apparatus is suited to examine a change of a blood flow characteristics in the brain triggered by a stimulation. These areas may thereby be interpreted as activated or deactivated areas.

The invention will now be described with reference to the accompanying drawings.

Figure 1      shows a navigation system for brain surgeries according to an aspect of the present invention;

Figure 2      shows an evaluation method according to an embodiment of the present invention;

Figure 3      shows a method for displaying a field of a brain of a patient according to an aspect of the present invention;

Figure 4      shows principles of a measuring process according to an embodiment of the present invention;

Figure 5      shows a field of a brain of a patient, wherein

Figure 5A      shows a white light image and

Figure 5B      shows a laser-Doppler-image;

Figure 6      shows a diagram according to an analysis method according to a preferred embodiment; and

Figure 7A and Figure 7B      show a superposition of a white light image and an output image gained according to an embodiment of a method of the present invention, wherein the output image is obtained from laser-Doppler-images acquired before and after a stimulation.

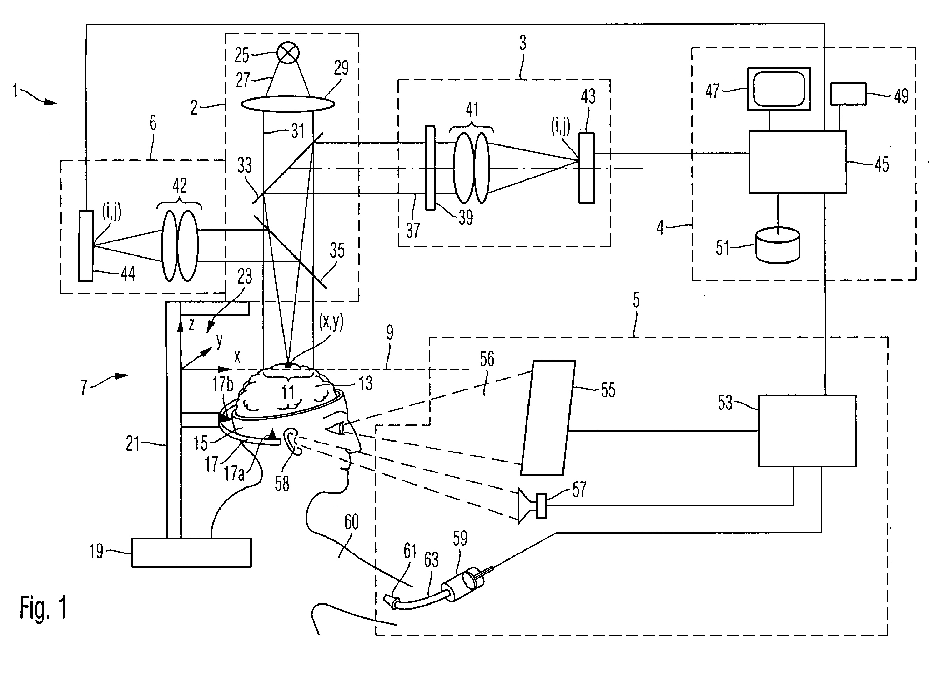

Figure 1 shows an embodiment of a navigation system for brain surgeries according to an aspect of the present invention. The navigation system 1 comprises an imaging system 2, a measuring light camera 3, a control and evaluation unit 4, a white light camera 6, a stimulation apparatus 5 and a position acquisition apparatus 7. In an object plane 9 of the imaging system 2 a field 11 of a brain 13 of a head 15 is arranged. The head 15 of the patient is connected to a base 19 using a head coupling apparatus 17 via an arrangement of levers 21. Thereby the head of the patient is held using pins 17a, 17b and a not illustrated pin 17c which are connected to the head coupling apparatus. A coordinate system 23 is connected to the base 19 and thus also to the head coupling apparatus 17. This coordinate system 23 is defined by coordinate axis x, y and z. The imaging system 2 is also connected via the arrangement of levers 21 to the base 19. The imaging system 2 comprises a light source 25 emitting light 27. In the embodiment illustrated here the light source is a laser emitting light having a wavelength of 808 nm. The coherence length of the light amounts to some mm. The light 27 emanating from the light source 25 is shaped by a beam shaping optics 29 to form measuring light 31 substantially comprising plane wave fronts. Measuring light 31 traverses a semi-transparent mirror 33 and a semi-transparent mirror 35. Subsequently the measuring light is incident onto the field 11 of the brain 13. Measuring light having interacted at a location x, y in the field 11 of the brain 13 emanates from this location x, y, traverses the semi-transparent mirror 35 and is reflected at the semi-transparent mirror 33 to form light 37. Light 37 enters the measuring light camera 3. Thereby the light 37 traverses a band-pass filter 39. The band-pass filter allows light within a wavelength range of 780 nm to 820 nm to pass. Subsequently the filtered light 37 traverses a camera objective 41 to be detected at a pixel (i,j) of a detector 43. The detector 43 comprises CMOS-sensors and is connected to the control and evaluation unit 4. The control and evaluation unit comprises a processing unit 45, a display unit 47, an input unit 49 and a storage unit 51. The processing unit 45 is adapted to evaluate a sequence of images each of which is given by image values I(i,j). The detailed evaluation method is described further below. The white light camera comprises a camera objective and a detector 44 for acquiring light in the visible wavelength range. A white light source not illustrated in Figure 1 illuminates the field 11 of the brain. White light reflected at a location (x, y) of the field of the brain is reflected at the semi-transparent mirror 35, traverses a camera objective and is detected at the pixel (i,j) of the detector 44. The white light image detected by the detector is displayed at the display unit 47.

The navigation system for brain surgeries further comprises a stimulation apparatus 5 for stimulating a patient. The simulation apparatus comprises a stimulation controller 43 as well as a variety of apparatuses for stimulating the patient. These apparatuses comprise in this embodiment a display unit 55, a speaker 57 and an injection robot 59. The display unit 55 is arranged in a field of view 56 of the patient. Also, the speaker 59 is within earshot of an ear 58 of the patient. At one arm 60 of the patient an infusion valve 61 is connected to a blood vessel of the patient. A supply tube 63 is inserted into the infusion valve. Via the supply tube 63 the injection robot is enabled to apply a variety of agents into the vascular system of the patient.

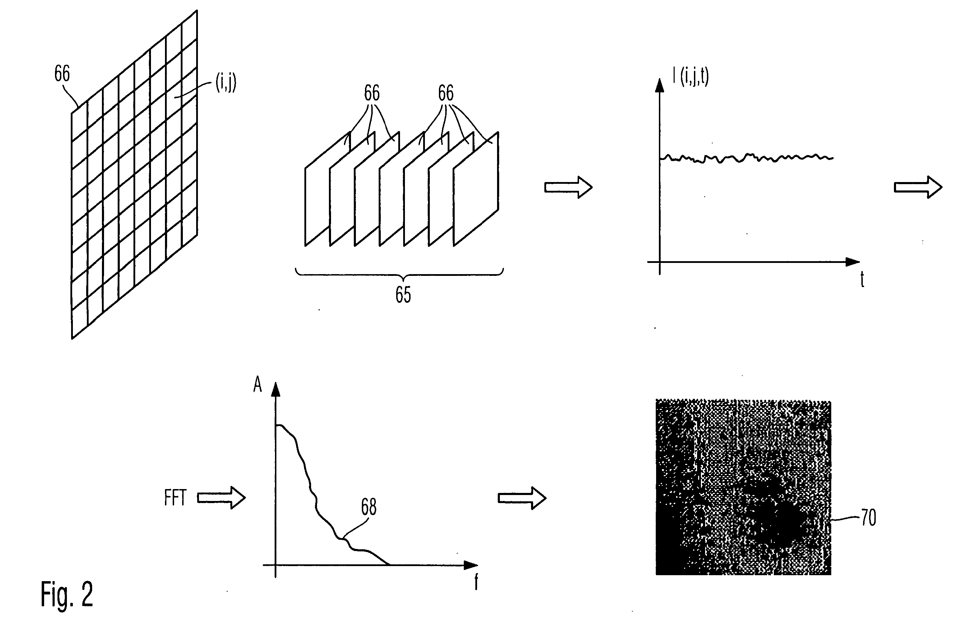

With reference to Figure 2 an evaluation method according to an embodiment of the present invention is illustrated. In this method a sequence 65 of images 66 is acquired by the detector 43 of Figure 1. In the illustrated embodiment every image 66 comprises a quadratic field of pixels (i,j).

The number of pixels in this embodiment is 512 x 512 pixels. Each pixel (i,j) of each image 66 of the sequence of images 65 is associated by the acquisition to an image value I (i,j). Thus, for each pixel (i,j) a temporal course of image values I (i,j,t) is established. The here illustrated method comprises an analysis of the temporal changes of these image values. In the diagram shown here (upper right in Figure 2) the image values are composed of a constant part not changing over time and a fluctuation part representing a change of the image values over time. Then in the method, a frequency spectrum of the temporal course of the image values is computed. The frequency spectrum may for example be obtained by a Fourier transformation. In particular, in the present embodiment, the FFT (fast Fourier transform) is employed. In the lower left part of Figure 2 the thus obtained frequency spectrum S(v) of the temporal changes of the image values is depicted and indicated by reference number 68. On the X-axis the frequency f is depicted and on the Y-axis the amplitude A of the respective frequency component is depicted. The frequency spectrum S(v) is commonly also referred to as power spectrum. It represents the amplitudes and frequencies of the temporally changing image values I (i,j,t). Then a first moment of the power spectrum is computed to compute for every pixel of the sequence 65 of images an analysis image value. This first moment is also referred to as a perfusion. Alternatively to the perfusion also a concentration C, or a velocity V of particles moving relative to each other may be computed as analysis image value according to the following formulas:

$$\text{Perfusion} = M_1 = \int_0^\infty vS(v)dv,$$

$$C = M_0 = \int_0^\infty vS(v)dv,$$

$$V = \frac{M_1}{M_0},$$

$$S(v) = \left| \int_0^\infty I(t)\,exp(-i\,2\pi vt)\,dt \right|^2.$$

If this method is carried out for all pixels in the sequence 65 of images, an analysis image 70 is obtained.

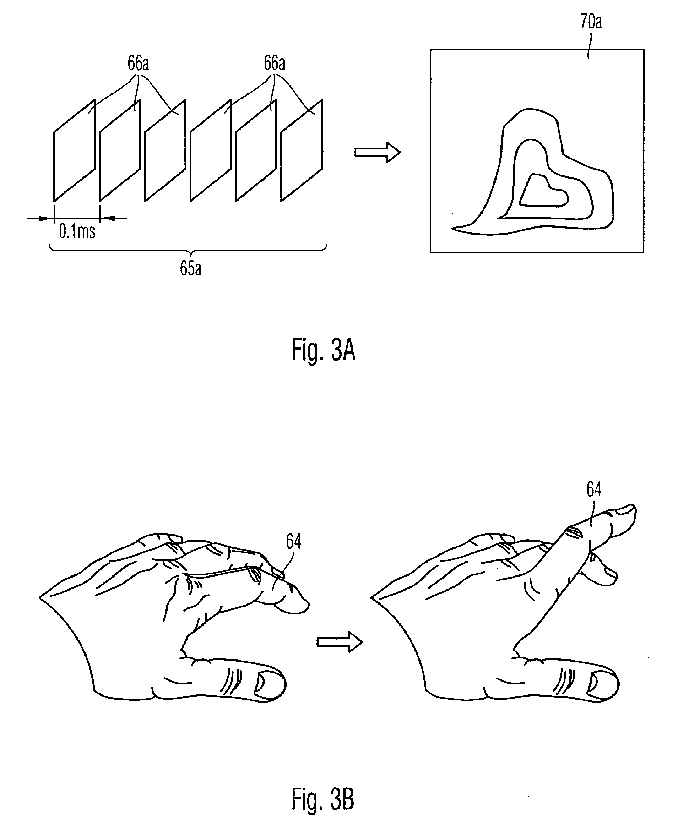

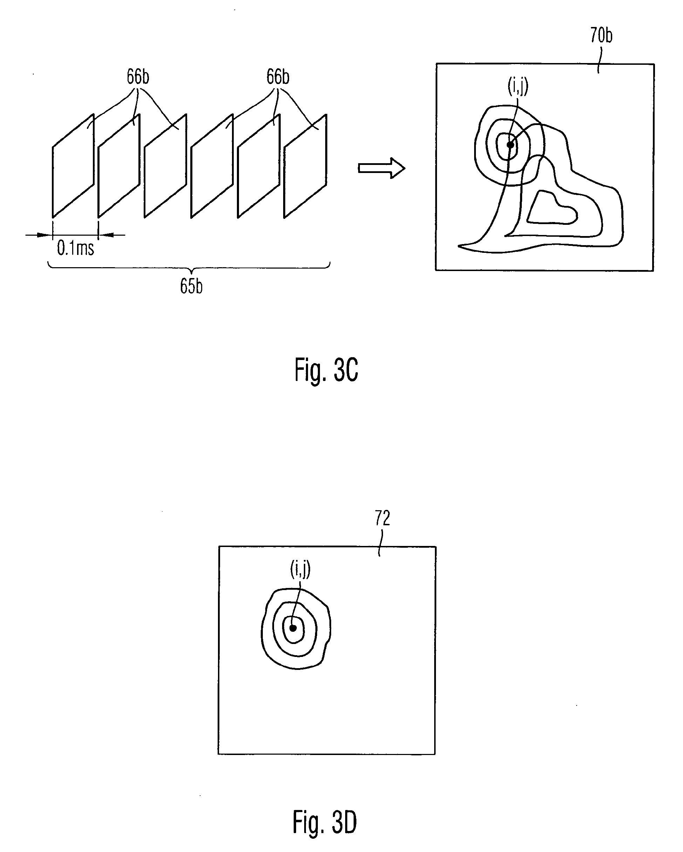

Figure 3 shows an embodiment of a method according to the invention. Thereby, a field of a brain of a patient is arranged in the object plane 9 of the imaging system 2 of the navigation system 1 for brain surgeries. The scull of the patient is opened to allow measuring light 31 to be incident onto a field 11 of the brain of the patient. In Figure 3A first a first sequence 65a of images 66a is acquired. Thereby the images 66a are acquired having a temporal interval of less than 0.1 ms. The acquired first sequence 65a of images 66a is then evaluated according to the evaluation method with reference to Figure 2. Thus, a first analysis image 70a is obtained representing information about a blood flow within the field 11 of the brain 13.

In Figure 3B the patient is then stimulated. In the illustrated embodiment the stimulating comprises a request to lift a finger 64. On the right hand side in Figure 3B it is shown that the patient follows the request and lifts the finger 64.

In Figure 3C then a second sequence 65b of images 66b of the field of the brain of the patient is acquired. Again, a temporal interval between acquisitions of subsequent images 66b is smaller than 0.1 ms. As in the case of the first sequence of images the second sequence of images 66b is evaluated to obtain a second analysis image 70b. Then an output image 72 is computed from a computation involving the first and the second analysis image, as illustrated in Figure 3D. In the example illustrated here the computing comprises forming a difference between the first and the second analysis image. The analysis image 72 is illustrated using contour lines in Figure 3D. It is apparent that in a region around the pixel (i,j) the output image 72 exhibits increased values. These increased values around the pixel (i,j) may indicate that areas around the corresponding location (x,y) of the field of the brain of the patient have been activated or deactivated by the stimulating illustrated in Figure 3B.

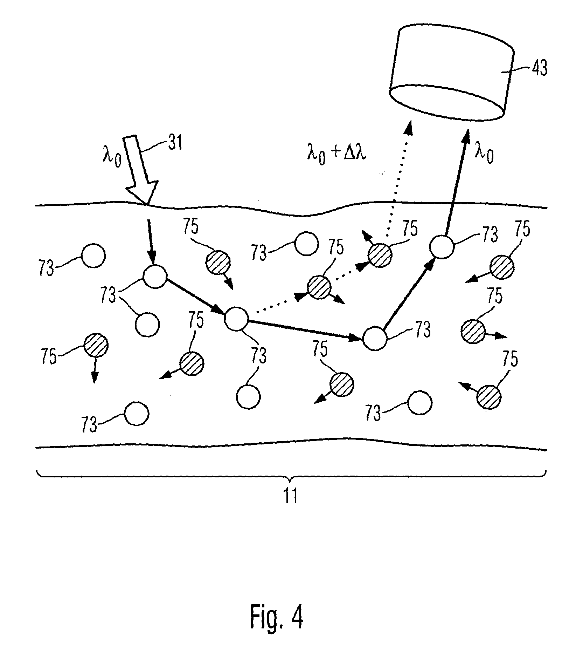

Figure 4 illustrates principles of the measurement according to an embodiment of the present invention. Light 31 is incident onto a field 11 of a brain of a patient. In this embodiment the light has a wavelength of 808 nm and is generated by a laser. A penetration depth of light of this wavelength into brain tissue amounts to about 1 mm to 2 mm. The spectral width of the light 31 having wavelength 880 nm generated by the laser is chosen such that a coherence length of the light results which lies in the range of the penetration depth of the light. Depending on the tissue structures present in the examined field 11 of the brain the penetration depth may amount to more or less than 1 mm to 2 mm. In the field 11 of the brain the incident light 31 is incident onto blood cells 73 and blood cells 75. Blood cells 73 (illustrated as circles having a white filling) are substantially at rest with respect to the examined field 11 of the brain. In contrast blood cells 75 (illustrated as circles having a striped filling) move relative to the examined field 11 of the brain according to a certain velocity whose magnitude and direction is indicated by arrows originating at the respective blood cell in Figure 4. The light 31 is reflected at the blood cells 73 and 75 and is scattered. While blood cells 73 being at rest relative to the field 11 reflect light 31 as light having the same wavelength $\lambda_0$ as the one of the incident light 31, blood cells 75 moving relative to the field 11 reflect the incident light 31 with wavelengths $\lambda_0 + \Delta\lambda$ different from the wavelengths of the incident light. If blood cells 75 move in a direction of the incident light 31, they reflect light with a wavelength which is greater than the wavelength of the incident light 31. If blood cells 75 move in a direction opposite to the incoming light 31, they reflect the light with wavelengths which are smaller than the wavelengths of the incident light. A plurality of blood cells 73 und 75 are hit by the incident light 31. Thus, the light reflected from this plurality of blood cells comprises, dependent on the velocity of the plurality of blood cells, wavelengths which are greater and smaller than the wavelength of the incident light. This velocity dependent shift of the wavelengths of the light is commonly referred to as Doppler-Effect. Light having different wavelengths and different relative phases superimposes, to be detected at a pixel of the detector 43. A superposition of light having slightly different wavelengths results in a temporally fluctuating signal which may also be denoted as beat signal. From the beat signal the different wavelengths generating this beat signals can be inferred. From the different wavelengths then the different velocities of the blood cells may be inferred which have generated the detected light by reflection. Using this method thereby information about a blood motion can be obtained. In particular, this method is very simple and cost effective. A further great advantage of this method is a high time resolution which lies in the range of below one second. Thus, in particular changes of a blood motion due to external influences or stimulations may be examined. Thus it is possible, to identify functional areas in the brain of a human. Identified areas in the brain may also be accounted for during brain surgeries. In particular functional areas may be circumvented to gain access to a region of the brain to be removed which region lies in deeper layers of the brain. The area to be removed may in particular be a tumour in the brain of the patient.

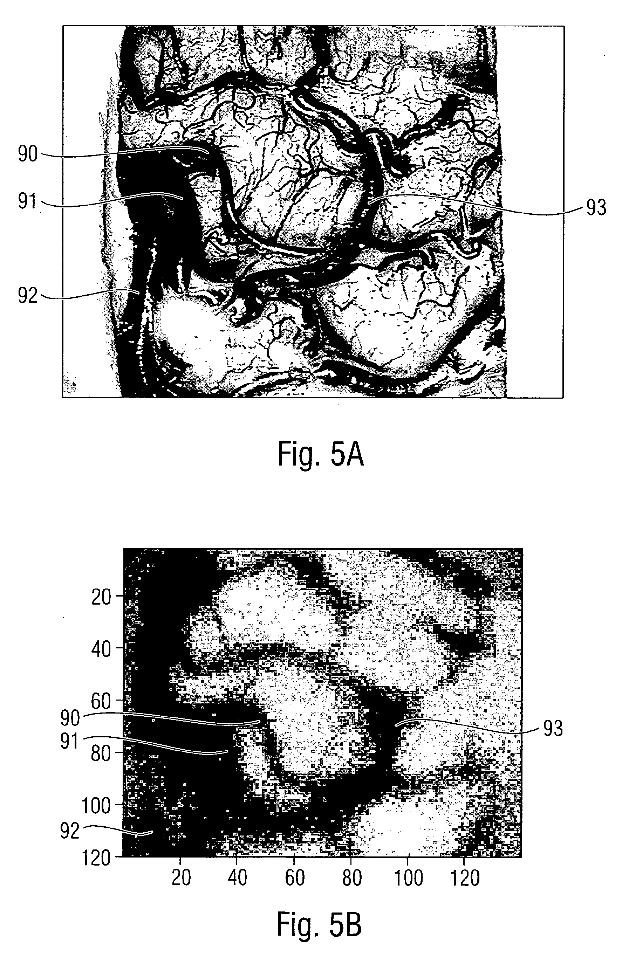

Figure 5A shows a white light image 74 of a field of a brain of a patient, wherein the patient's skullcap is opened. On the surface of the brain of the patient several blood vessels 76 are visible. These blood vessels comprise arteries and veins. The blood vessels branch out more and more to continue into micro capillaries 78. Between the blood vessels white brain tissue is visible.

Figure 5B shows a laser-Doppler-image 70 acquired from the same field of the brain of the patient. Higher values of a blood flow, respectively perfusion, are depicted by brighter grey values. One recognizes a correspondence of structures in the white light image 74 and the laser-Doppler-image 70. In particular it is apparent that areas of the field of the brain which show blood vessels in the white light image indicate in the laser-Doppler-image a rather decreased blood motion. A possible explanation may be that a blood motion in the blood vessels apparent in the white light image 74 is too fast that it may not anymore be acquired using the embodiment of the measuring apparatus illustrated here. Indeed blood flow velocities in aortas amount to up to some meters per second. Blood flow velocities decrease in middle sized and smaller capillaries to some centimetres per second. Only in very small micro capillaries blood flow velocities of some millimetres per second are found which may be detected by the measuring apparatus of the present embodiment. In the embodiment the white light image 74 and the laser-Doppler-image 70 are obtained by simultaneous illumination with laser light and white light and simultaneous detection.

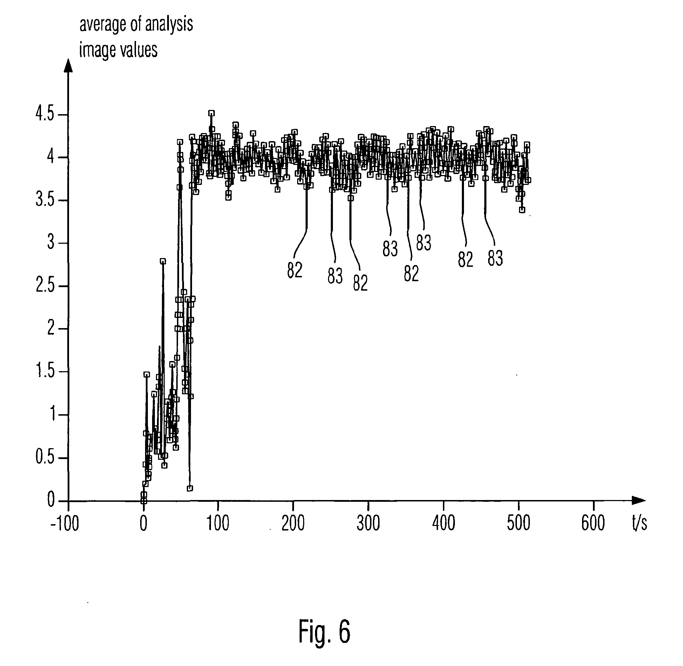

Figure 6 shows a diagram depicting an average of analysis image values of all pixels of a laser-Doppler-image in dependence of time t in units of seconds s. Thus, the diagram in Figure 6 represents an average blood motion in a field of a brain of a human over time. In this example the patient was requested at the time points 82 to count loudly. At the time points denoted by reference number 83 the patient was requested to stop counting. This sequence of requests was repeatedly performed, wherein the requests for counting and for stopping the counting were performed for different time spans. One recognizes the dependency of the intensity signal depicted on the y-axis from the time points 82 of the request to count and the time points 83 of the requests to stop counting. Thus, it is shown that it is possible using the inventive method to detect changes in a blood flow in the brain due to stimulations.

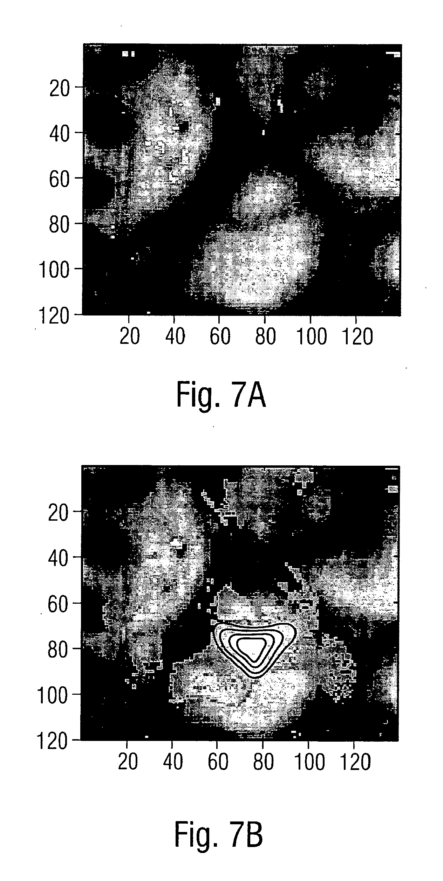

Figures 7A and 7B show output images according to the method for displaying a field of a brain superimposed with white light images of the same field of the brain. The numbers along the image edges indicate pixel indices. The white light image thereby is illustrated as grey scale image in Figures 7A and 7B, respectively. The output image is illustrated as contour line image. The output image was obtained in the following way: First and second sequences of images were acquired, wherein the patient moved a finger during the acquisition of the second sequence of images. Thereby eight sequences of images were acquired, where upon the patient moved the finger and alternating thereto eight sequences of images were acquired, where upon the patient kept the finger calmly. Thereby the durations of the sequences were not uniformly but amounted sometimes to 20 s, sometimes to 10 s, and sometimes to 30 s. The output images depicted in Figure 7A and 7B were obtained by comparison of the average value of the images of the first sequences with the average value of the images of the second sequences. While contour lines in the image of Figure 7A indicate an increase of a perfusion or a blood motion in the field of the brain, contour lines in the image of Figure 7B indicate a decrease of the perfusion or the blood motion of the field of the brain due to the moving a finger. It is apparent that in a central lower area of the acquired field of the brain a pronounced decrease of the blood motion was detected during the moving the finger. In other areas of the acquired field of the brain such detected decreases or increases of a blood motion continuous over a certain area do not occur. A possible interpretation of this finding is that the continuous area of the field of the brain identified by a significant decrease is activated or deactivated during moving a finger. Conversely also a finger movement may occur due to an activation or deactivation of this area. Interestingly, exactly this area was identified from independent electro stimulation experiments as an area which is related to a finger movement. Other areas shown in Figures 7A and 7B were not detected as areas which are related to a finger movement. Thus, the inventive method proves to be a non-invasive method to identify functional areas in the brain of a human.

**Claims**

1. A method for displaying a field (11) of a brain (13) of a patient, the method comprising:

    illuminating the field (11) of the brain (13) with measuring light and acquiring at least one first sequence (65a) of images (66a) of the illuminated field,
    then stimulating the patient,
    then illuminating the field (11) of the brain with measuring light (31) and acquiring at least one second sequence (65b) of images (66b) of the illuminated field, and
    evaluating the first sequence (65a) and the second sequence (65b) of images by:

    (a) associating a first analysis image value ($70a(i,j)$) to pixels ($i,j$) in the first sequence (65a) of images ($I(i,j,t)$) to which pixels same locations ($x,y$) of the field (11) are imaged, respectively, wherein the first analysis image value depends on at least one of amplitudes and frequencies of temporal changes of image values ($I(i,j,t)$) of these pixels ($i,j$),
    (b) associating a second analysis image value ($70b(i,j)$) to pixels ($i,j$) in the second sequence (65b) of images ($I(i,j,t)$) to which pixels the same locations ($x,y$) of the field (11) are imaged, respectively, wherein the second analysis image value depends on at least one of amplitudes and frequencies of temporal changes of image values ($I(i,j,t)$) of these pixels ($i,j$), and

    wherein the method further comprises:

    determining an output image (72) from the first ($70a(i,j)$) and the second analysis image values ($70b(i,j)$) and displaying the output image (72).

2. The method according to claim 1, wherein the acquiring the images of the first and the second sequence comprises:

    exposing detector elements of an image detector (43) during an exposure time of less than 1 ms, in particular less than 0.1 ms, and further in particular less than 0.03 ms.

3. The method according to claim 1 or 2, wherein the acquiring the images of the first and the second sequence comprises acquiring temporally subsequent images having a temporal interval of less than 1 ms, in particular less than 0.1 ms, and further in particular less than 0.05 ms.

4. The method according to one of claims 1 to 3, further comprising establishing a coordinate system (23) relative to a head (15) of the patient.

5. The method according to one of claims 1 to 4, further comprising determining at least one continuous region in the output image (72) in which intensity values are greater than a threshold.

**6.** The method according to one of claims 1 to 5, wherein at least one of the first analysis image value and the second analysis image value represents a value which comprises at least one of a perfusion ($M_1$), a concentration ($M_0$), an average velocity (V), a parameter for a velocity distribution, in particular a standard deviation of the velocity distribution of particles moving relative to each other, in particular blood cells (73, 75).

**7.** The method according to one of the preceding claims, wherein the stimulating comprises requesting the patient to move a particular body part (64).

**8.** The method according to one of the preceding claims, wherein the stimulating comprises applying at least one of a visual stimulus, an acoustic stimulus, a gestation stimulus, an olfaction stimulus, and a tactition stimulus to the patient.

**9.** The method according to one of the preceding claims, further comprising: acquiring an image (74) of the field (11) of the brain (13) in a visible wavelength range.

**10.** The method according to claim 9, further comprising superimposing at least two of the image in the visible wavelength range, the first analysis image values, the second analysis image values, and the output image on a display.

**11.** A navigation system for brain surgery, comprising:

a position acquisition apparatus to acquire coordinates of locations of a brain of a patient, and
a measuring apparatus for acquiring images, wherein the measuring apparatus is configured:

to illuminate a field (11) of the brain (13) with measuring light (31) and to acquire at least one sequence (65) of images (66) of the illuminated field (11), and
to process the sequence (65) of images (66) by associating an analysis image value (70(i,j)) to pixels (i,j) in the sequence of images (I(i,j,t)) to which pixels same locations (x,y) of the field (11) are imaged, respectively, wherein the analysis image value depends on at least one of amplitudes and frequencies of temporal changes of image values (I(i,j,t)) of these pixels (i,j).

**12.** The navigation system according to claim 11, wherein the measuring apparatus for acquiring the images comprises a camera (3) having an exposure integration time of less than 1 ms, in particular less than 0.1 ms, and further in particular less than 0.03 ms.

**13.** The navigation system according to claim 11 or 12, wherein the measuring apparatus for acquiring the images comprises a camera (3) having a frame rate greater than 1 kHz, in particular greater than 10 kHz, and further in particular greater than 20 kHz.

**14.** The navigation system according to one of claims 11 to 13, wherein a band-pass filter (39) is arranged in a beam path upstream of the camera (41, 43), wherein a transmission characteristics of the band-pass filter (39) is adapted such that a transmission of light having wavelengths greater than 1300 nm and smaller than 600 nm, in particular greater than 820 nm and smaller than 780 nm, amounts to less than 10 % of a total transmission of the band-pass filter.

**15.** The navigation system according to one of claims 11 to 14, wherein the measuring apparatus comprises a light source (25) having an emission spectrum formed such that an emitted intensity of light having wavelengths greater than 1300 nm and smaller than 600 nm, in particular greater than 820 nm and smaller than 780 nm, amounts to less than 10 % of a total emitted intensity.

**16.** The navigation system according to one of claims 11 to 15, further comprising an apparatus (6) for acquiring images of the brain of the patient in a visible wavelength range.

**17.** The navigation system according to claim 16, further comprising a display apparatus (47) for displaying an image in the visible wavelength range, in particular in a superposition with analysis image values.

**18.** The navigation system according to one of claims 11 to 17, wherein the system is adapted to perform the method according to one of claims 1 to 10.

Fig. 1

Fig. 2

EP 1 982 645 A1

Fig. 3A

Fig. 3B

66b    66b

0.1ms

65b

70b

(i,j)

**Fig. 3C**

72

(i,j)

**Fig. 3D**

Fig. 4

Fig. 5A

Fig. 5B

Fig. 6

Fig. 7A

Fig. 7B

**European Patent Office**

## PARTIAL EUROPEAN SEARCH REPORT

which under Rule 63 of the European Patent Convention shall be considered, for the purposes of subsequent proceedings, as the European search report

Application Number

EP 08 00 7595

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/39873 A (APPLIED SPECTRAL IMAGING LTD [IL]; GIL AMIR [IL]; GIL TAMIR [IL]; HORN) 23 May 2002 (2002-05-23)<br>* page 44, lines 5-28 *<br>* page 45, line 4 - page 46, line 2 *<br>* page 77, lines 9-16 *<br>* page 84, line 8 - page 85, line 4 *<br>* figure 6 * | 11,16-18 | INV.<br>A61B5/00<br>A61B19/00 |
| Y | | 14,15 | |
| | ----- | | |
| X | TAKASHIMA I ET AL: "High-speed CCD imaging system for monitoring neural activity in vivo and in vitro, using a voltage-sensitive dye"<br>JOURNAL OF NEUROSCIENCE METHODS, ELSEVIER SCIENCE PUBLISHER B.V., AMSTERDAM, NL,<br>vol. 91, 15 September 1999 (1999-09-15), pages 147-159, XP002419199<br>ISSN: 0165-0270<br>* paragraph [2.1.1] *<br>* paragraph [2.3.1] *<br>* paragraph [02.6] *<br>* paragraph [003.] *<br>* table 1 * | 11-13,16 | |
| | | | **TECHNICAL FIELDS SEARCHED (IPC)**<br><br>A61B |
| | ----- | | |
| | -/-- | | |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 12 August 2008 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent
Office

**PARTIAL EUROPEAN SEARCH REPORT**

Application Number

EP 08 00 7595

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | WO 93/25141 A (UNIV WASHINGTON [US]) 23 December 1993 (1993-12-23) * page 18, lines 1-16 * * page 19, lines 9-14 * * page 26, line 1 - page 27, line 19 * * page 41, line 5 - page 42, line 8 * ----- | 11,16-18 | |
| Y | US 5 215 095 A (MACVICAR BRIAN A [CA] ET AL) 1 June 1993 (1993-06-01) * column 5, lines 55-66 * * figure 1 * ----- | 14,15 | |
| A | DATABASE INSPEC [Online] THE INSTITUTION OF ELECTRICAL ENGINEERS, STEVENAGE, GB; 15 June 2004 (2004-06-15), ZEPEDA A ET AL: "Optical imaging of intrinsic signals: recent developments in the methodology and its applications" XP002491866 Database accession no. 8268859 * the whole document * & Journal of Neuroscience Methods Elsevier Netherlands, vol. 136, no. 1, 15 June 2004 (2004-06-15), pages 1-21, ISSN: 0165-0270 ----- | 11-18 | TECHNICAL FIELDS SEARCHED (IPC) |

EPO FORM 1503 03.82 (P04C10)

European Patent
Office

**INCOMPLETE SEARCH
SHEET C**

Application Number

EP 08 00 7595

Claim(s) not searched:
        1-10

Reason for the limitation of the search (non-patentable invention(s)):

Article 53 (c) EPC - Method for treatment of the human or animal body by surgery

## ANNEX TO THE EUROPEAN SEARCH REPORT
## ON EUROPEAN PATENT APPLICATION NO.

EP 08 00 7595

This annex lists the patent family members relating to the patent documents cited in the above-mentioned European search report.
The members are as contained in the European Patent Office EDP file on
The European Patent Office is in no way liable for these particulars which are merely given for the purpose of information.

12-08-2008

| Patent document cited in search report | | Publication date | Patent family member(s) | | Publication date |
|---|---|---|---|---|---|
| WO 0239873 | A | 23-05-2002 | AU | 2398902 A | 27-05-2002 |
| WO 9325141 | A | 23-12-1993 | AT | 222725 T | 15-09-2002 |
| | | | AU | 666569 B2 | 15-02-1996 |
| | | | AU | 4532493 A | 04-01-1994 |
| | | | AU | 5227896 A | 11-07-1996 |
| | | | CA | 2136634 A1 | 23-12-1993 |
| | | | DE | 69332244 D1 | 02-10-2002 |
| | | | DE | 69332244 T2 | 04-12-2003 |
| | | | EP | 0644737 A1 | 29-03-1995 |
| | | | ES | 2184742 T3 | 16-04-2003 |
| | | | JP | 7507472 T | 24-08-1995 |
| | | | JP | 3848329 B2 | 22-11-2006 |
| | | | JP | 2004255180 A | 16-09-2004 |
| US 5215095 | A | 01-06-1993 | CA | 2048697 A1 | 11-02-1992 |
| | | | US | 5438989 A | 08-08-1995 |

EPO FORM P0459

For more details about this annex : see Official Journal of the European Patent Office, No. 12/82